# EUROPEAN PATENT APPLICATION

(11) **EP 3 736 330 A1**
(43) Date of publication of application: **11.11.2020**
(21) Application number: 19173365.8
(22) Date of filing: 08.05.2019
(51) Int. Cl.: C12N 7/00, C12N 15/86, A61K 48/00

(54) **MODIFIED ADENO-ASSOCIATED VIRUS (AAV) PARTICLES FOR GENE THERAPY**

(71) Applicant: European Molecular Biology Laboratory, 69117 Heidelberg (DE)
(72) Inventor: HEPPENSTALL, Paul, 00015 Monterotondo (IT); MAFFEI, Mariano, 00198 Rome (IT); DE CASTRO REIS, Fernanda, 00015 Monterotondo (IT); POUW, Kanyn Morris, 3961 DN Wijk bij Duurstede (NL)
(74) Representative: Krauss, Jan

(57) **Abstract**

The present invention relates to improved adeno-associated virus (AAV) particles for gene delivery and gene therapy. Provided are adeno associated virus (AAV) particles that comprise a modified capsid. The present invention further relates to methods for producing the improved AAV particles of this invention by removing natural binding sites in adeno associated virus (AAV) capsids and introducing ligand binding sites into said capsid to provide AAVs that transduce only particular cells of interest. An additional aspect of the present invention relates to modified AAV particles for use in the treatment of a disease and methods for treating a disease, comprising administering the modified AAV particles to a subject in need thereof. Yet a further aspect of this invention relates to the AAV particles of this invention for the transfection of cells, for example as a gene delivery tool in basic research.

## Description

The present invention relates to improved adeno-associated virus (AAV) particles for gene delivery and gene therapy. Provided are adeno associated virus (AAV) particles that comprise a modified capsid. The present invention further relates to methods for producing the improved AAV particles of this invention by removing natural binding sites in adeno associated virus (AAV) capsids and introducing ligand binding sites into said capsid to provide AAVs that transduce only particular cells of interest. An additional aspect of the present invention relates to modified AAV particles for use in the treatment of a disease and methods for treating a disease, comprising administering the modified AAV particles to a subject in need thereof. Yet a further aspect of this invention relates to the AAV particles of this invention for the transfection of cells, for example as a gene delivery tool in basic research.

### Background of the invention

Introduction of molecules carrying genetic information into cells is a useful tool in modern medicine and in basic research. Preferred methods include the use of gene delivery vehicles derived from viruses, including adenoviruses, retroviruses, vaccina viruses, and adeno associated viruses. Among these, adeno-associated viruses (AAV) are preferred viruses for gene therapy methods. The wild type AAVs are DNA viruses of relatively small size which integrate, in a stable and site-specific manner into the genome of the cells they infect. Importantly, so far no human disease has been found to be associated with AAV infection. Thus, Adeno-associated virus (AAV) has become the gene therapy vector of choice due to its lack of pathogenicity. Interestingly, more than 100 clinical trials are underway using AAV-based vectors, and, importantly, the first AAV gene therapy was recently FDA approved, namely Voretigene neparvovec for the treatment of an inherited retinal disease.

Adeno-associated viruses are members of the genus Dependovirus of the Parvoviridae family containing a non-enveloped icosahedral capsid. The AAV genome is approximately 4.7 kilobases long and is composed of linear single-stranded deoxyribonucleic acid (ssDNA), which may be either positive- or negative-sensed. The genome comprises inverted terminal repeats (ITRs) at both ends of the DNA strand, and two open reading frames (ORFs): rep and cap. The rep frame is made of four overlapping genes encoding non-structural replication (Rep) proteins required for the AAV life cycle. The cap frame contains overlapping nucleotide sequences of structural VP capsid proteins: VP1, VP2 and VP3, which interact together to form a capsid of an icosahedral symmetry. The terminal 145 nt are self-complementary and are organized so that an energetically stable intramolecular duplex forming a T-shaped hairpin may be formed. These hairpin structures function as an origin for viral DNA replication, serving as primers for the cellular DNA polymerase complex. Following AAV infection in mammalian cells the rep genes (i.e. Rep78 and Rep52) are expressed from the P5 promoter and the P19 promoter, respectively, and both Rep proteins have a function in the replication of the viral genome. A splicing event in the rep ORF results in the expression of four Rep proteins (i.e. Rep78, Rep68, Rep52 and Rep40).

In general, viruses can enter the cell in a variety of ways, either through direct membrane fusion/penetration reactions at the plasma membrane, or by undergoing endocytosis followed by a similar breaching of the cell membrane in an early endosome. The most common form of viral internalization is through clathrin-mediated endocytosis. This process can be separated into several steps, which include: (1) the nucleation of a clathrin coated pit; (2) cargo capture in coated pits; (3) curvature induction and membrane invagination; and (4) vesicle scission and uncoating. The vesicle delivers its viral content to early endosomes. Interestingly, the entire process of endocytosis takes place on the order of seconds *in vivo.* Examples for viruses that are internalized via clathrin-mediated endocytosis are dsDNA viruses, such as Adenoviridae, Adenovirus type 2, Adenovirus type 5, Adenovirus type 8, Adenovirus type 37, canine adenovirus type 2 (CAV-2), Adeno-associated virus 2 and Adeno-associated virus 5 (Dependovirus).

Viruses can also be internalized via caveolae, which are specialized lipid rafts that form 50-70 nm flask-shaped invaginations of the plasma membrane. Caveolins form the structural backbone of caveolae. The internalization via caveolae is not a constitutive process but only occurs upon cell stimulation. Internalized viruses bound to their host cell receptor are delivered to the early endosome. Caveolae represent a low capacity but highly regulated pathway. Furthermore, some viruses are internalized using several pathways that do not use a clathrin or caveolin coat and that are sometimes hijacked by bacteria and viruses to gain access to the host cell. These pathways can be further defined by their dependency to various molecules such as cholesterol, DNM2/Dynamin-2, or small GTPases or tyrosine kinase.

Because of their stable and site-specific integration into the genome of infected cells and their lack of pathogenicity, AAV vectors are being explored as vehicles for targeted gene therapy.

Kern et al. (in: Identification of a Heparin-Binding Motif on Adeno-Associated Virus Type 2 Capsids, Journal of Virology Sep 2003, 77 (20) 11072-11081) disclose that infection of cells with adeno-associated virus (AAV) type 2 (AAV-2) is mediated by binding to heparan sulfate proteoglycan and can be competed by heparin. Mutational analysis of AAV-2 capsid proteins showed that a group of basic amino acids (arginines 484, 487, 585, and 588 and lysine 532) contribute to heparin and HeLa cell binding. These amino acids are positioned in three clusters at the threefold spike region of the AAV-2 capsid. The tissue distribution in mice of recombinant AAV-2 mutated in R484 and R585 indicated markedly reduced infection of the liver, compared to infection with wild-type recombinant AAV, but continued infection of the heart. They suggested that although heparin binding influences the infectivity of AAV-2, it seems not to be necessary.

US 5,756,283 discloses recombinant AAV vectors comprising a selected transgene under the control of regulatory sequences. The infected cell is contacted with an agent that facilitates the conversion of single stranded recombinant virus to its double stranded form, thereby enhancing the efficiency of transduction of the recombinant AAV into the target cell.

EP 1664314 B1 describes recombinant AAV vectors carrying capsid protein modification(s) resulting in a reduced or eliminated heparin binding function, based on the identification of a capsid protein domain and corresponding amino acid residues involved in heparin binding.

WO 2017/143100 A1 describes methods to modify AAV capsid polypeptides to exhibit an enhanced neutralization profile, increased transduction and/or tropism in human liver tissue or hepatocyte cells, as compared to non-variant parent capsid polypeptides.

Safety and efficacy of human gene therapy continue to be the subject of considerable debate. Problems of current vectors include unintended transduction of certain tissues, and adverse immune reactions. Current approaches of using AAV particles for gene delivery are problematic due to the lack of efficient transduction, i.e. very high titers of AAV vectors are usually needed to be effective. Another limitation of current approaches is that many AAV vectors are ineffective at transducing some specific cell types and AAV vectors can have off-target effects through transduction of inappropriate cell types.

In view of the above limitations, it is an underlying task of this invention to provide improved adeno associated virus (AAV) particles for gene delivery. The above is achieved by engineering an adeno associated virus (AAV) capsid that transduces specific cells of interest and/or that can be delivered at a lower titre but still be effective. The above problem is solved by modifying a virus capsid to accept a ligand attachment and attaching a ligand of interest to said capsid. Optionally, natural binding sites in the AAV capsid can be removed prior to modifying the virus to accept the ligand attachment.

According to a first aspect of the present invention, the above object is solved by providing an adeno associated virus (AAV) particle, comprising a modified capsid, wherein said modified capsid comprises at least one modification selected from the removal of a natural binding site in said capsid, and the introduction of a ligand binding site into said capsid.

The terms "adeno-associated viral vector", "AAV vector", "adeno-associated virus", "AAV virus", "AAV virion", "AAV viral particle" and "AAV particle", that shall be used interchangeably herein, refer to a viral particle composed of at least one AAV capsid protein and an encapsidated recombinant viral genome. An AAV particle that comprises a recombinant viral genome having a heterologous polynucleotide and a transcriptional regulatory region that comprises a promoter to be delivered to a mammalian cell flanked by the AAV inverted terminal repeats is typically referred to as an "AAV vector particle" or an "AAV vector".

In one preferred embodiment, the adeno associated virus (AAV) particle of the present invention is selected from AAV1, AAV2, AAV3, AAV4, AAV5, AAV6, AAV7, AAV8, AAV9, AAV10, AAV11, and AAV12. The most commonly used gene transfer systems to date are derivatives of viruses, e.g. adeno-associated virus type 2 (AAV-2).

Currently, there are more than 100 AAV serotypes identified that differ in the binding capacity of capsid proteins to specific cell surface receptors that can transduce different cell types. AAV2 was the first serotype cloned into a bacterial plasmid and has since been used as a comparison to identify other serotypes. Twelve serotypes (AAV1, AAV2, AAV3, AAV4, AAV5, AAV6, AAV7, AAV8, AAV9, AAV10, AAV11, and AAV12) have been tested thoroughly for their ability to transduce specific cell types and differentiated between capsid protein motifs that bind specific cell surface receptors for cell attachment. In the context of this invention, an AAV particle selected from AAV1, AAV2, AAV3, AAV4, AAV5, AAV6, AAV7, AAV8, AAV9, AAV10, AAV11, AAV12 is preferred. However, it should be understood that any other AAV particle can be used in the context of the present invention.

The capsid of AAV is composed of three overlapping capsid proteins (VP1, VP2, VP3) containing a unique VP1 N-terminus, a VP1/VP2 common portion and a portion which is common to VP1, VP2 and VP3.

An adeno associated virus (AAV) particle of the present invention is further preferred, wherein at least one protein in said capsid of AAV particle is modified, preferably wherein at least one protein is VP1, VP2, and/or VP3. Alternatively, two of the proteins VP1, VP2 and/or VP3 in said capsid are modified, or all three of the proteins VP1, VP2 and VP3 in said capsid are modified. Preferably, at least one part, e.g. one amino acid, of the at least one of the proteins to be modified in said capsid is modified. However, it is also possible to modify multiple parts of the proteins VP1, VP2 and VP3 in said capsid, e.g. multiple amino acids, such as 2, 3, 4, 5, 6, 7, 8, 9, 10, or any other number of parts or amino acids. Preferably at least one of arginines 484, 487, 585 and 588 and lysine 532 of VP1 and/or an analogous arginine in VP2 or VP3 are removed by replacing them with a different amino acid, such as alanine.

Further preferred is that the capsid modification comprises both the removal of at least one natural binding site and the introduction of at least one ligand binding site. Alternatively, the at least one natural binding site of said AAV can stay unchanged, i.e. not be removed, but the at least one ligand binding site is introduced.

If said natural binding site in said capsid is present and not removed, and at least one additional ligand binding site is introduced into said capsid by modification, the AAV particle of this invention has a higher infectivity rate at lower titers of the virus particle as used. Contrary, if said natural binding site in said capsid is removed prior to introducing at least one additional ligand binding site into said capsid by modification, the tropism of the AAV particle of this invention is modified.

Preferred is the adeno associated virus (AAV) particle of the present invention, wherein said natural binding site is a binding site that enables binding to heparan sulfate proteoglycans, and is preferably removed by replacing at least one of arginine 585 or arginine 588 of VP1 and/or an analogous arginine in VP2 or VP3 with a different amino acid, such as alanine.

Additionally preferred is an adeno associated virus (AAV) particle of the present invention, wherein said ligand binding site is a binding site that enables the covalent attachment of a ligand. Further preferred is that said ligand binding site comprises a benzylguanine group, a benzylcytosine group, a chloroalkane group, an azide group, a phosphine, or combinations thereof. Alternatively, any haloalkane group can be used. Additionally preferred is that the benzylguanine or other group is attached to an available lysine residue. Said ligand binding site as introduced is preferably attached to the ε-amino group or the primary amine of said available lysine residue.

Further preferred is an adeno associated virus (AAV) particle of the present invention, further comprising a ligand that is attached to said benzylguanine group, said benzylcytosine group, said chloroalkane group, said azide group, and/or said phosphine in particular a HaloTag™, a *SNAP-tag™* or *a* CLIP-tag™, or a phosphine or an azide. The present invention preferably utilizes tags that are able to bind to their specific ligands with high affinity, such as SNAP-tag, CLIP-tag, Halo Tag, Lumio Tag, and others.

Benzylguanine, benzylcytosine and chloroalkane are recognized by a "suicide" enzyme, such as SNAP. In the context of this invention, benzylguanine, or benzylcytosine derivatives can further be used. Benzylguanine derivatives or benzylcytosine derivatives are understood to mean a benzylguanine or benzylcytosine group, which is modified but which is nevertheless recognized by the suicide enzyme.

The tag molecule may be any molecule or biomolecule, which is capable of specifically binding to a further molecule. The examples may include SNAP-tag, CLIP-tag, Lumio-Tag, or Halo-Tag. For example, the affinity tag may be a SNAP-tag, a mutant of an alkylguanine-DNA alkyltransferase. Importantly, one of the substrates for SNAP-tag is benzylguanine. Commercially available products useful for the present invention include, e.g., HaloTag from Promega, Lumio Tag from Life Technologies, and SNAP/CLIP Tags from NEB.

The person of skill is well aware of further methods for attaching a ligand to a capsid, such as anti-tag antibodies, streptavidin-biotin, or chemical crosslinking. Any of the known methods can be used in the context of the present invention. For example, unnatural amino acids could be incorporated in the capsid as well as in the ligand. Subsequently, crosslinkers, such as bioorthogonal crosslinkers could be used to covalently attach the ligand to the capsid. In a further example a phosphine could be incorporated in the capsid and an azide in the ligand or vice versa. The ligand could then be covalently attached to the capsid via the Staudinger reaction.

Any kind of ligand can be attached to said ligand binding site. Preferably, the ligand to be attached is selected from a protein ligand, such as a growth factor or a cytokine; a toxin subunit, such as a cholera toxin B subunit; a lectin, such as isolectin B4 or wheat germ agglutinin; an adhesion factor, such as lactadherin; an antibody, such as an anti CD-34 antibody; a peptide, such as deltorphin opioid receptor ligand; and a gene editing nuclease, such as Cas9.

Nucleic acid molecules packaged inside the adeno associated virus (AAV) particle of the present invention can be any kind of nucleic acid molecule. Preferably, said nucleic acid molecule is encoding intracellular antibodies (for example to neutralize certain proteins inside cells), nucleic acid molecules encoding peptide toxins (for example to block ion channels in the pain pathway), nucleic acid molecules encoding optogenetic actuators (for example to turn on or turn off neuronal activity using light), nucleic acid molecules encoding pharmacogenetic tools (for example to turn on or off neuronal signaling using chemical ligands that have no interfering pharmacological effect), nucleic acid molecules encoding CRISPR based-editors for precision gene editing, nucleic acid molecules encoding CRISPR-epigenetic tools to regulate gene expression, and/or nucleic acid molecules encoding suicide genes to induce cell death.

Preferably, when the ligand is a gene editing nuclease, such as Cas9, the adeno associated virus (AAV) particle of the present invention further carries a nucleic acid molecule as a cargo, such as a gRNA and/or a specific DNA to be inserted into a host genome.

The person of skill is aware of other gene editing nucleases, apart from Cas9, such as Cpfl, TALEN, ZFN, or a homing endonuclease. Further, it may be convenient to engineer using DNA-guided Argonaute interference systems (DAIS). Basically, said Argonaute (Ago) protein is heterologously expressed from a polynucleotide introduced into said cell in the presence of at least one exogenous oligonucleotide (DNA guide) providing specificity of cleavage to said Ago protein to a preselected locus. The TALEN and Cas9 systems are respectively described in WO 2013/176915 and WO 2014/191128. The Zinc-finger nucleases (ZFNs) are initially described in Kim, YG; Cha, J.; Chandrasegaran, S. ("Hybrid restriction enzymes: zinc finger fusions to Fok I cleavage domain" (1996). Proc Natl Acad Sci USA 93 (3): 1156-60). Cpfl is a class 2 CRISPR Cas System described by Zhang et al. (Cpfl is a single RNA-guided Endonuclease of a Class 2 CRIPR-Cas System. (2015). Cell;163:759-771). The argonaute (AGO) gene family was initially described in Guo S, Kemphues KJ. (Par-1, a gene required for establishing polarity in C. elegans embryos, encodes a putative Ser/Thr kinase that is asymmetrically distributed. (1995). Cell;81(4):611-20).

Another aspect of this invention then relates to the AAV particle according to the present invention for use in the treatment of a disease.

Any kind of disease can be treated or prevented by the AAV particle for use according to the present invention. Preferably, diseases to be treated or prevented by the AAV particle for use according to the present invention are diseases that can be treated by gene therapy, such as cancer, an inherited monogenic disease, such as inherited retinal disease, a genetic skin disease, such as Olmsted Syndrome or Familiar Primary Localized Cutaneous Amyloidosis, an infectious disease, adrenoleukodystrophy, alpha-1 antitrypsin deficiency, aromatic L-amino acid deficiency, Batten disease, Becker muscular dystrophy, beta thalassemia, Canavan disease, chronic granulomatous disease, Crigler-Najjar syndrome, cystic fibrosis, Duchenne muscular dystrophy, Fabry disease, familial adenomatous polyposis, familial hypercholesterolaemia, familial lecithin-cholesterol acyltransferase deficiency, Fanconi anaemia, galactosialidosis, Gaucher's disease, gyrate atrophy, hemophilia A and B, Hurler syndrome (mucopolysaccharidosis type I), Hunter syndrome (mucopolysaccharidosis type II), Huntington's chorea, junctional epidermolysis bullosa, late infantile neuronal ceroid lipofuscinosis, leukocyte adherence deficiency, limb girdle muscular dystrophy, lipoprotein lipase deficiency, metachromatic leukodystrophy, Sly syndrome (mucopolysaccharidosis type VII), Netherton syndrome, ornithine transcarbamylase deficiency, Pompe disease, purine nucleoside phosphorylase deficiency, recessive dystrophic epidermolysis bullosa, sanfilippo A (mucopolysaccharidosis type IIIA), sanfilippo B (mucopolysaccharidosis type IIIB), sickle cell disease, severe combined immunodeficiency, spinal muscular atrophy, Tay Sachs disease, Wiskott-Aldrich syndrome, von Gierke disease (glycogen storage disease type Ia), X-linked myotubular myopathy, anemia of end stage renal disease, angina pectoris (stable, unstable, refractory), coronary artery stenosis, critical limb ischemia, heart failure, intermittent claudication, myocardial ischemia, peripheral vascular disease, pulmonary hypertension, venous ulcers, adenovirus infection, cytomegalovirus infection, Epstein-Barr virus infection, hepatitis B infection, hepatitis C infection, HIV/AIDS, influenza, Japanese encephalitis, malaria, pediatric respiratory disease, respiratory syncytial virus, tetanus, tuberculosis, gynaecological cancer, breast, ovary, cervix, vulva, nervous system cancer, glioblastoma, leptomeningeal carcinomatosis, glioma, astrocytoma, neuroblastoma, retinoblastoma, gastrointestinal cancer, colon, colorectal, liver metastases, post-hepatitis liver cancer, pancreas, gall bladder, hepatocellular carcinoma, genitourinary cancer, prostate, renal, bladder, ano-genital neoplasia, skin cancer, melanoma (malignant/metastatic), head and neck cancer, nasopharyngeal carcinoma, squamous cell carcinoma, esophageal cancer, lung cancer, adenocarcinoma, small cell/non-small cell, mesothelioma, hematological cancer, leukemia, lymphoma, multiple myeloma, sarcoma, germ cell cancer, Li-Fraumeni syndrome, thyroid cancer, Alzheimer's disease, amyotrophic lateral sclerosis, carpal tunnel syndrome, chronic traumatic brain injury, cubital tunnel syndrome, diabetic neuropathy, epilepsy, giant axonal neuropathy, late infantile neuronal ceroid lipofuscinosis, multiple sclerosis, myasthenia gravis, pain, Parkinson disease, peripheral neuropathy, spinal muscular atrophy type 2, achromatopsia, age-related macular degeneration, choroideraemia, diabetic macular oedema, glaucoma, Leber congenital amaurosis, macular telangiectasia type 2, retinitis pigmentosa, superficial corneal opacity, X-linked retinoschisis, arthritis (rheumatoid, inflammatory, degenerative), degenerative joint disease, severe inflammatory disease of the rectum, ulcerative colitis, chronic renal disease, diabetic ulcer/foot ulcer, detrusor overactivity, erectile dysfunction, fractures, hearing loss, hereditary inclusion body myopathy, graft versus host disease/transplant patients, oral mucositis, parotid salivary hypofunction, systemic scleoderma, type I diabetes, and/or wound healing.

The term "preventing and/or inhibiting", as used herein, shall include treating an already existing disease. Treatment, prevention, and/or inhibition is meant to include, e.g., treating, delaying or alleviating disease progression, reducing the symptoms of, or curing the disease or condition. An "effective amount" is an amount of the AAV particle of the present invention that alleviates symptoms as found for the disease to be treated, such as any of the diseases named above. Alleviating is meant to include, e.g., preventing, treating, reducing the symptoms of, or curing the disease or condition. The invention also includes a method for treating a subject at risk for a development and/or progression of a disease, wherein a therapeutically effective amount of the AAV particle of the present invention of this invention is administered to the patient. Being at risk for the disease can result from, e.g., phenotypic symptoms, which predispose to the disease. As used herein, the term "prevention" or "preventing" when used in the context of a subject refers to stopping, hindering, and/or slowing down the development or onset of a disease, and in particular the symptoms associated with the disease.

Yet another embodiment of the invention pertains to the afore-described AAV particle for use in the treatment of a disease, wherein said AAV is administered to a subject in a liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray.

Another aspect of this invention then relates to a method of producing an improved adeno associated virus (AAV) particle, comprising the step of introducing at least one modification into a capsid of said AAV, preferably wherein said modification comprises introducing at least one ligand binding site into said capsid, optionally wherein a natural binding site in said capsid is removed, such as is previously removed.

As mentioned above, if said natural binding site in said capsid is present and not removed, and at least one additional ligand binding site is introduced into said capsid by modification, the adeno associated virus (AAV) particle of this invention has a higher infectivity rate at lower titers of the virus particle as used. Contrary, if said natural binding site in said capsid is removed prior of introducing at least one additional ligand binding site into said capsid by modification, the tropism of the adeno associated virus (AAV) particle of this invention is modified.

The adeno associated virus (AAV) particle produced by the above method is preferably selected from AAV1, AAV2, AAV3, AAV4, AAV5, AAV6, AAV7, AAV8, AAV9, AAV10, AAV11, AAV12, and mixtures thereof.

Any of the proteins of the adeno associated virus (AAV) particle to be produced by the above method can be modified. Preferably, at least one of the proteins VP1, VP2 or VP3 in said capsid is modified in the above method. Alternatively, two of the proteins VP1, VP2 and/or VP3 in said capsid are modified, or all three of the proteins VP1, VP2 and VP3 in said capsid are modified. Preferably, at least one part, e.g. at least one amino acid, of the at least one of the proteins to be modified in said capsid is modified. However, it is also possible to modify multiple parts of the proteins VP1, VP2 and VP3 in said capsid, e.g. multiple amino acids, such as 2, 3, 4, 5, 6, 7, 8, 9, 10, or any other number of parts or amino acids. Preferably at least one of arginines 484, 487, 585 and 588 and lysine 532 of VP1 and/or an analogous arginine in VP2 or VP3 are removed by replacing them with a different amino acid, such as alanine.

A protein, such as VP1, VP2 or VP3, may be modified chemically by reacting specific amino acids. Examples for such modifications are well known in the art and are summarized e.g. in R. Lundblad, Chemical Reagents for Protein Modification, 3rd ed. CRC Press, 2005, which is incorporated herein by reference. Chemical modification of amino acids includes but is not limited to, modification by acylation, amidination, pyridoxylation of lysine, reductive alkylation, trinitrobenzylation of amino groups with 2,4,6-trinitrobenzene sulphonic acid (TNBS), amide modification of carboxyl groups and sulphydryl modification by performic acid oxidation of cysteine to cysteic acid, formation of mercurial derivatives, formation of mixed disulphides with other thiol compounds, reaction with maleimide, carboxymethylation with iodoacetic acid or iodoacetamide and carbamoylation with cyanate at alkaline pH, although without limitation thereto. In this regard, the skilled person is referred to Chapter 15 of Current Protocols In Protein Science, Eds. Coligan et al. (John Wiley & Sons NY 1995-2000) for more extensive methodology relating to chemical modification of proteins.

The above method for producing an improved adeno associated virus (AAV) particle is preferred, wherein said capsid modification comprises both removing of natural binding sites and introducing of ligand binding sites. Alternatively, the natural binding site of said AAV can stay unchanged, i.e. not be removed, but at least one ligand binding site is introduced.

In a preferred embodiment said natural binding site is removed by the above method for producing an improved adeno associated virus (AAV) particle, wherein said binding site is a natural binding site that enables binding to heparan sulfate proteoglycans, and is preferably removed by replacing at least one of arginines 585 and 588 of VP1 and/or an analogous arginine in VP2 or VP3 with a different amino acid, such as alanine.

More preferred is that the ligand binding site as introduced is one that enables the covalent attachment of ligands, and is preferably selected from a benzylguanine group that is attached to available lysine residues, more preferably by reacting said capsid with benzylguanine N-hydroxysuccinimide (BG-NHS), and/or benzylcytosine N-hydroxysuccinimide (BC-NHS).

The present invention preferably utilizes tags that are able to bind to their specific ligands with high affinity, such as SNAP-tag, CLIP-tag, Halo-Tag, Lumio-Tag, and others. The tag molecule as introduced in the above-method may be any molecule or biomolecule, which is capable of specifically binding to a further molecule. The examples may include SNAP-tag, CLIP-tag, Lumio-Tag, or Halo-Tag. For example, the affinity tag may be a SNAP-tag, a mutant of an alkylguanine-DNA alkyltransferase. Importantly, one of the substrates for SNAP-tag is benzylguanine. Commercially available products useful for the present invention include, e.g., HaloTag from Promega, Lumio Tag from Life Technologies, and SNAP/CLIP Tags from NEB. Said ligand binding site as introduced is preferably attached to the ε-amino group or the primary amine of said available lysine residue.

Accordingly, the above method for producing an improved adeno associated virus (AAV) particle is further preferred, wherein said method further comprises the step of attaching a ligand to said benzylguanine and/or said benzylcytosine group, in particular a HaloTag™, a SNAP-tag™ or a CLIP-tag™.

Said ligand to be attached can be any kind of ligand, but is preferably selected from a protein ligand, such as a growth factor or a cytokine; a toxin subunit, such as a cholera toxin B subunit; a lectin, such as isolectin B4 or wheat germ agglutinin; an adhesion factor, such as lactadherin; an antibody, such as an anti CD-34 antibody; a peptide, such as deltorphin opioid receptor ligand; and a gene editing nuclease, such as Cas9.

A further aspect of this invention then relates to a method for treating a disease that can be treated by gene therapy, comprising administering the AAV particle according to this invention to a subject in need thereof.

In the context of the present invention, the term "subject", as used in certain embodiments, preferably refers to a mammal, such as a mouse, rat, guinea pig, rabbit, cat, dog, monkey, or preferably a human. The term "patient" preferably refers to a mammal, such as a mouse, rat, guinea pig, rabbit, horse, cattle, cow, cat, dog, monkey, or preferably a human, for example a human patient, for whom diagnosis, prognosis, or therapy is desired. The subject of the invention may be at danger of suffering from a disease, such as a bacterial infection, a viral infection, a fungal infection, or a parasitic infection. A more detailed description of medical indications relevant in the context of this invention is provided herein elsewhere.

Cells and/or subjects to be treated with the AAV particles of this invention are preferably of mammalian origin, such as of human origin. Nevertheless, the present invention can advantageously be used also in veterinary medicine, cell culture procedures, or even in plant cell diseases, depending on the similarities of the mechanisms of entry into the cells. Preferably, said cell to be treated is a mammalian cell, a prokaryotic cell, or a plant cell. Most preferably said cell to be treated is a human cell.

Yet another embodiment of the invention pertains to the afore-described method for treating a disease, comprising administering the AAV particle according to this invention to a subject in need thereof, wherein said AAV particle is administered to a subject in a liquid, dry or semi-solid form, such as, for example, in the form of a tablet, coated tablet, effervescent tablet, capsule, powder, granulate, sugar-coated tablet, lozenge, pill, ampoule, drop, suppository, emulsion, ointment, gel, tincture, paste, cream, moist compress, gargling solution, plant juice, nasal agent, inhalation mixture, aerosol, mouthwash, mouth spray, nose spray, or room spray.

The disease to be treated by the above method for treating a disease that comprises administering the AAV particle to a subject, is preferably a disease selected from cancer, an inherited monogenic disease, such as inherited retinal disease, a genetic skin disease, such as Olmsted Syndrome or Familiar Primary Localized Cutaneous Amyloidosis, an infectious disease, adrenoleukodystrophy, alpha-1 antitrypsin deficiency, aromatic L-amino acid deficiency, Batten disease, Becker muscular dystrophy, beta thalassemia, Canavan disease, chronic granulomatous disease, Crigler-Najjar syndrome, cystic fibrosis, Duchenne muscular dystrophy, Fabry disease, familial adenomatous polyposis, familial hypercholesterolemia, familial lecithin-cholesterol acyltransferase deficiency, Fanconi anemia, galactosialidosis, Gaucher's disease, gyrate atrophy, hemophilia A, hemophilia B, Hurler syndrome (mucopolysaccharidosis type I), Hunter syndrome (mucopolysaccharidosis type II), Huntington's chorea, junctional epidermolysis bullosa, late infantile neuronal ceroid lipofuscinosis, leukocyte adherence deficiency, limb girdle muscular dystrophy, lipoprotein lipase deficiency, metachromatic leukodystrophy, Sly syndrome (mucopolysaccharidosis type VII), Netherton syndrome, ornithine transcarbamylase deficiency, Pompe disease, purine nucleoside phosphorylase deficiency, recessive dystrophic epidermolysis bullosa, sanfilippo A (mucopolysaccharidosis type IIIA), sanfilippo B (mucopolysaccharidosis type IIIB), sickle cell disease, severe combined immunodeficiency, spinal muscular atrophy, Tay Sachs disease, Wiskott-Aldrich syndrome, von Gierke disease (glycogen storage disease type Ia), X-linked myotubular myopathy, anemia of end stage renal disease, angina pectoris (stable, unstable, refractory), coronary artery stenosis, critical limb ischemia, heart failure, intermittent claudication, myocardial ischemia, peripheral vascular disease, pulmonary hypertension, venous ulcers, adenovirus infection, cytomegalovirus infection, Epstein-Barr virus infection, hepatitis B infection, hepatitis C infection, HIV/AIDS, influenza, Japanese encephalitis, malaria, pediatric respiratory disease, respiratory syncytial virus, tetanus, tuberculosis, gynecological cancer, breast cancer, ovary cancer, cervix cancer, vulva cancer, nervous system cancer, glioblastoma, leptomeningeal carcinomatosis, glioma, astrocytoma, neuroblastoma, retinoblastoma, gastrointestinal cancer, colon, colorectal, liver metastases, post-hepatitis liver cancer, pancreas, gall bladder, hepatocellular carcinoma, genitourinary cancer, prostate, renal, bladder, ano-genital neoplasia, skin cancer, melanoma (malignant/metastatic), head and neck cancer, nasopharyngeal carcinoma, squamous cell carcinoma, esophageal cancer, lung cancer, adenocarcinoma, small cell/non-small cell, mesothelioma, hematological cancer, leukemia, lymphoma, multiple myeloma, sarcoma, germ cell cancer, Li-Fraumeni syndrome, thyroid cancer, Alzheimer's disease, amyotrophic lateral sclerosis, carpal tunnel syndrome, chronic traumatic brain injury, cubital tunnel syndrome, diabetic neuropathy, epilepsy, giant axonal neuropathy, late infantile neuronal ceroid lipofuscinosis, multiple sclerosis, myasthenia gravis, pain, Parkinson disease, peripheral neuropathy, spinal muscular atrophy type 2, achromatopsia, age-related macular degeneration, choroideraemia, diabetic macular edema, glaucoma, Leber congenital amaurosis, macular telangiectasia type 2, retinitis pigmentosa, superficial corneal opacity, X-linked retinoschisis, arthritis (rheumatoid, inflammatory, degenerative), degenerative joint disease, severe inflammatory disease of the rectum, ulcerative colitis, chronic renal disease, diabetic ulcer, foot ulcer, detrusor overactivity, erectile dysfunction, fractures, hearing loss, hereditary inclusion body myopathy, graft versus host disease/transplant patients, oral mucositis, parotid salivary hypofunction, systemic scleoderma, type I diabetes, and wound healing, or combinations thereof.

Further preferred is a method for treating a disease, comprising administering the AAV particle according to this invention to a subject in need thereof, wherein said AAV particle is administered to said subject or to a cell in form of a pharmaceutical composition, e.g. in combination with pharmaceutically acceptable additives, carriers, diluents, solvents, filters, lubricants, excipients, binders or stabilizers. Preferably, said composition is administered to said subject in form of sprays, coatings, foams, lotions, gels, mouthwash, oral formulations or injections. Said composition can be administered to said subject systemically, orally or by any other clinically/medically accepted method.

A further aspect of the present invention then relates to a pharmaceutical composition, comprising the AAV particle according to the present invention, together with at least one pharmaceutically acceptable carrier and/or diluent, i.e. in combination with pharmaceutically acceptable additives, carriers, diluents, solvents, filters, lubricants, excipients, binders or stabilizers. Preferably, said composition is administered to said subject in form of sprays, coatings, foams, lotions, gels, mouthwash, oral formulations or injections. Said composition can be administered to said subject systemically, orally or by any other clinically/medically accepted method.

A further aspect of the present invention then relates to a kit comprising:
a) the AAV particle as disclosed and/or for use according to this invention, or a pharmaceutical composition comprising the AAV particle as disclosed according to this invention,
b) written instructions to apply said AAV particle or said pharmaceutical composition to a target said; and
optionally, a container holding the AAV particle for use or the composition and the written instructions.

Another aspect of the present invention relates to the use of the above-described kit for preventing, treating, and/or inhibiting a viral infection in a subject in need of said treatment.

Yet another aspect of this invention relates to the AAV particle according to this invention for use in the transfection of a cell, for example as a gene delivery tool in research. Said use can also be for cosmetic purposes, and the present invention includes a method for cosmetic treatment in analogy to the medical treatment as disclosed herein. For this, administering the AAV particle according to this invention to a subject or to a cell can be also achieved in form of a cosmetic composition, e.g. in combination with cosmetically safe and acceptable additives, carriers, diluents, solvents, filters, lubricants, excipients, binders or stabilizers. Preferably, said composition is administered to said subject in form of sprays, coatings, foams, lotions, gels, mouthwash, oral formulations or injections. Said composition can be administered to said subject systemically, orally or by any other clinically/cosmetically accepted method.

The person of skill is aware of methods of using vectors derived from AAV for transferring genes in vitro and in vivo, such as those that have been described in WO 93/09239, US4797368, US 5139941 and EP 488 528.

An additional aspect of the present invention relates to a kit comprising:
a) the AAV particle for the transfection of cells,
b) written instructions to use the AAV particle for the transfection of cells; and
optionally, a container holding the AAV particle and the written instructions.

Preferred features of each aspect of the invention are as for each of the other aspects mutatis mutandis. The prior art documents mentioned herein are incorporated to the fullest extent permitted by law. Although the present invention and its advantages have been described in detail, it should be understood that various changes, substitutions and alterations can be made herein without departing from the spirit and scope of the invention as defined in the appended claims.

The present invention will be further illustrated in the following examples and figures, which are given for illustration purposes only and are not intended to limit the invention in any way. For the purpose of the present invention, all references as cited are hereby incorporated by reference in their entireties.
Figure 1 shows a schematic depiction of SNAP-tagged ligands with the BG-modified virus.
Figure 2 shows that the ΔHSPG virus particle according to the invention has no more infective activity (dark picture); the construct was tested on sensory neurons in a fluorescent reporter mouse model. The inset shows the phase contrast microscopic image of the cells.
Figure 3 shows that the wheat germ agglutinin (WGA) fusion fully reverted the viral transduction efficiency to 100% (fluorescent cells) when tested on sensory neurons in a fluorescent reporter mouse model in analogy to Figure 2.
Figure 4 shows that neurotrophic factors NGF (A), NT3 (B) and BDNF (C) deliver virus to different neuronal populations. The insets show the microscopic image of the cells, the constructs were tested on sensory neurons in a fluorescent reporter mouse model in analogy to Figure 2.
Figure 5 shows cholera toxin B subunit transported virus retrogradely to neuronal cell bodies when injected into the skin. The inset shows the microscopic image of the cells, the construct was tested on sensory neurons in a fluorescent reporter mouse model in analogy to Figure 2.
Figure 6 shows sensory neuron tissue in the trigeminal ganglia three weeks after injection with a virus with NGF ligand IV according to the invention (A), stained with an antibody against TrkA (the receptor for NGF, B). An overlap of at least 80% can be seen (C).
Figure 7 shows a staining of the sections from Figure 6 with antibodies against NF200 and IB4, which mainly mark other neurons (mechanoreceptors (green/grey) and non-peptidergic nociceptors, respectively (blue/dark grey)). The red (light grey) infected cells are mainly different from the green and blue cells.

### Examples

The aim of the experiments as performed in the context of the present invention was to engineer the adeno associated virus (AAV) capsid so that the virus will transduce only cells of interest. This was achieved by removing the natural binding sites for cells in the native AAV capsid protein(s). The modified virus is then suitably (in particular chemically) modified in order to accept a selective controlled ligand attachment. These desired ligands are then covalently attached to the virus, and tested in vitro on cells, and in vivo in mice.

### 1. Removal of natural binding sites in AAV2

AAV2 binds to Heparan Sulfate Proteoglycans through arginine 585 and 588. These positions were mutated to alanine to create the deletion ΔHSPG.

The Plasmid pTAV2-0 contains the entire AAV-2 genome from pAV-2, including both inverted terminal repeats, cloned into the *Bam*HI site of pBluescript II. A sub-plasmid containing a suitable fragment of the AAV-2 was created and used as the template for site-directed mutagenesis reactions. Mutagenesis was performed by using a Stratagene (Amsterdam, The Netherlands) QuikChange site-directed mutagenesis kit according to the manufacturer's protocol. For each mutant, two complementary PCR primers were designed to contain the sequence of the substitution, flanked by 15 to 20 homologous base pairs on each side of the mutation. Mutant plasmids were identified by DNA sequencing. The fragment containing the suitable mutation was then subcloned into a plasmid backbone (e.g. pTAV2-0), containing the rest of the protein, and the complete fragment was sequenced to check for additional PCR mutations.

### 2. Chemical modification of ΔHSPG for accepting ligands

Typically, selective attachment of ligands to proteins, e.g. protein labeling, is accomplished by incorporation of bioorthogonal groups into a protein, followed by chemoselective modifications. This approach is also designated as "tag-and-modify". A variety of bioorthogonal reactions have been developed, which can be classified into: (1) condensation reactions through carbonyls, (2) "click" reactions through azides, (3) inverse electron-demand Diels-Alder cycloadditions (DA_{INV}) and other cycloaddition reactions, (4) transition metal-catalyzed coupling and decaging reactions, and (5) labeling reactions at cysteine residues. Benzylguanine (BG) was subsequently attached to exposed lysine by reacting virus with benzylguanine NHS ester (SNAP tag substrate or BG--NHS). For this, using a needle, nonaqueous DMSO was added to the vial with the dry SNAP tag ligand BG-NHS to the desired final concentration (e.g. 20 mM) at room temperature. The protein to be amine-functionalized was diluted in solvent (PBS) to the desired final concentration. The two preparations were mixed and incubated at room temperature for 180 minutes, followed by removal of the unreacted components using a centrifugal 100Kda MWCO filter unit.

### 3. Covalent attachment of ligands

There are two steps to using this system: cloning and expression of the protein of interest as a SNAP-tag® fusion, and labeling of the fusion with the SNAP-tag substrate of choice. The SNAP-tag is a small protein based on human O⁶-alkylguanine-DNA-alkyltransferase (hAGT), a DNA repair protein. The SNAP-tag substrate in this case is the guanine leaving group connected to a benzyl linker. In the labeling reaction, the substituted benzyl group of the substrate is covalently attached to the SNAP-tag.

The SNAP-tag protein labeling system enables the specific, covalent attachment of virtually any molecule to a protein of interest (for the present invention, see 4., below).

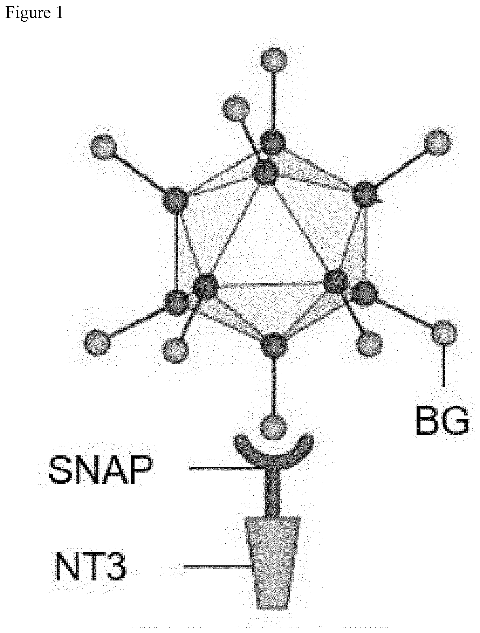

Recombinant ligands with C terminal SNAP tags were produced in E. coli or in mammalian cells in suspension culture. For the covalent attachments, SNAP-tagged ligands were then attached to the BG-modified virus (see Figure 1) by adding saturating concentrations of ligand and incubating at room temperature overnight. Excess non-reacted ligand was removed by passing the reaction through a centrifugal 100Kda MWCO filter unit.

For the present invention, the experiments were performed in accordance according with the instructions of the SNAP-Cell® Starter Kit (NEB) containing a mammalian expression plasmid (pSNAP_{f)} encoding the SNAP-tag® flanked by restriction sites for cloning a gene of interest, with modifications for the present purpose.

### 4. In vitro and in vivo tests

In the context of the present invention, the above strategy was tested with multiple classes of ligands, namely protein ligands, like growth factors, cytokines etc.; toxin subunits, like cholera toxin B subunit; lectins, such as isolectin B4 or wheat germ agglutinin; adhesion factors, like lactadherin; antibodies, such as anti CD-34 (marker of stem cells); and peptides, such as deltorphin opioid receptor ligand.

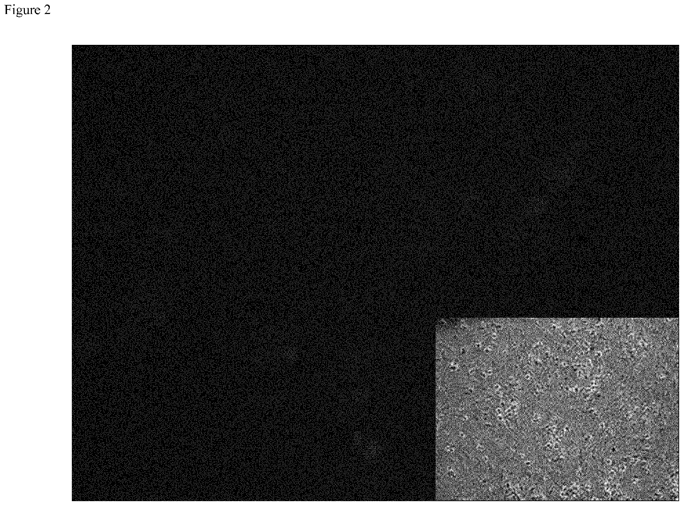

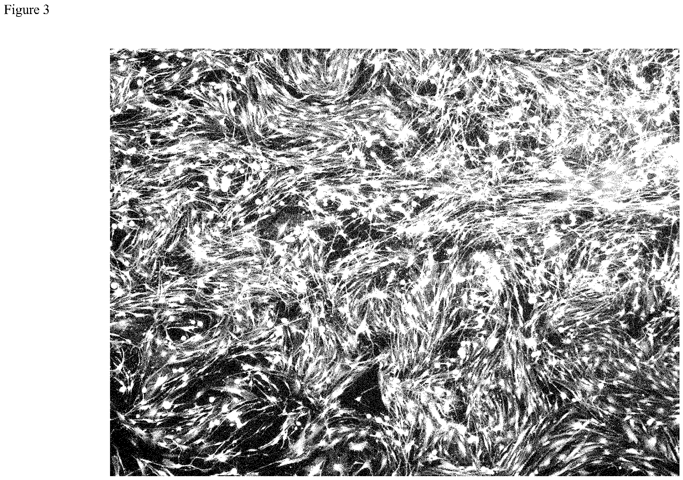

It was shown first that the ΔHSPG virus particle according to the invention had no more infective activity as tested on sensory neurons in a fluorescent reporter mouse model (Figure 2). The wheat germ agglutinin (WGA, lectin) fusion fully reverted the viral transduction efficiency to 100% when tested on sensory neurons in the same fluorescent reporter mouse model (Figure 3).

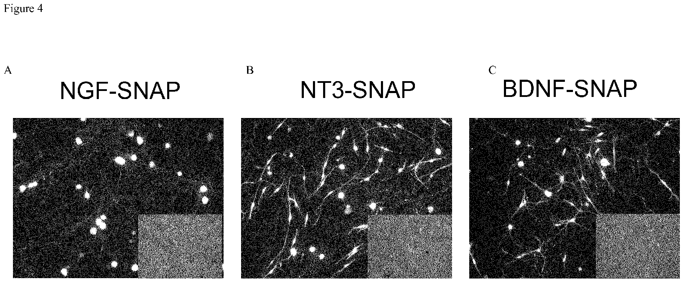

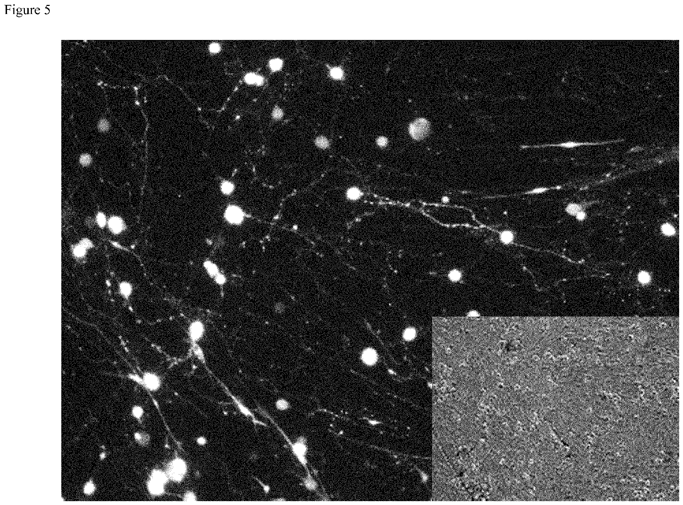

Then, several factors were tested, the neurotrophic factors NGF, NT3 and BDNF (protein ligands) delivered virus to different specific neuronal populations depending on the factor used in a fluorescent reporter mouse model (Figure 4). Cholera Toxin B subunit (toxin) specifically directed virus retrogradely to neuronal cell bodies (i.e. cell compartment/part specific) (Figure 5). In similar tests, lactadherin (adhesion factor) specifically directed virus to macrophages and neurons exposing phosphatidylserine, and deltorphin (peptide) specifically directed virus to neurons expressing the Mu and Delta opioid receptors.

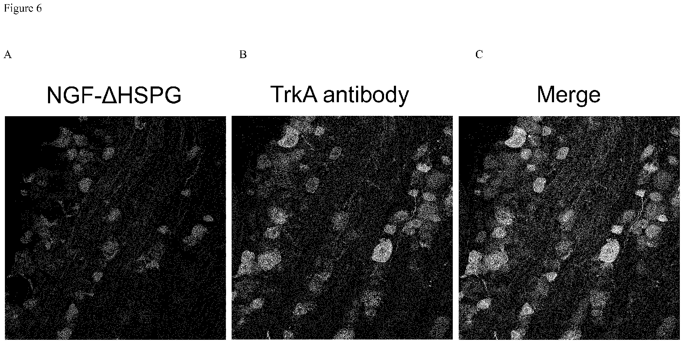

In the experiments shown in Figure 6, virus with the NGF ligand IV was injected into the trigeminal ganglia, then sensory neuron tissue was taken and analysed three weeks later. The sectioned tissue was stained with an antibody against TrkA (the receptor for NGF), and a very good overlap was found. The TrkA antibody is not perfect, so an 80% overlap is extremely relevant.

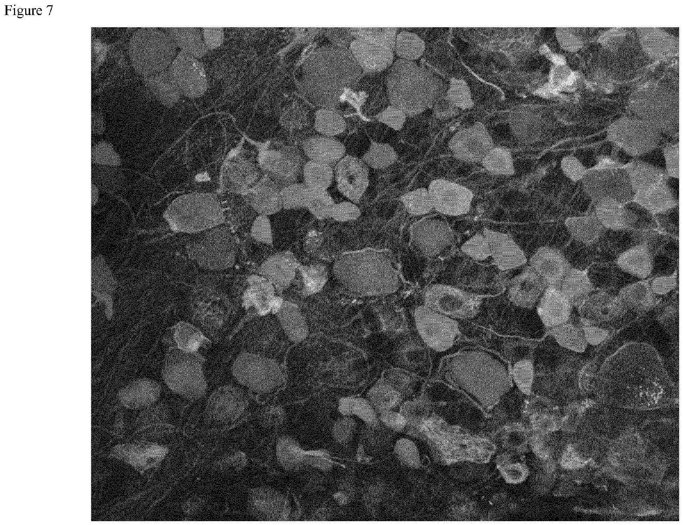

In the experiments shown in Figure 7, the sections from Figure 6 were stained with antibodies against NF200 and IB4, which label other neurons (mechanoreceptors and non-peptidergic nociceptors, respectively). Again, these markers are not perfect but it can be seen that the green and blue cells are different from the red infected cells.

As a negative control, virally introducing the IL31 ligand into an IL31 receptor knockout mouse does not lead to an infection.

In summary, all ligand-labeled viruses successfully and specifically transduced only those cells expressing the respective receptor, both when applied in vitro to cultured cells, and when injected in vivo in mice, i.e. can be injected systemically or locally and selectively target different populations of cells.

## Claims

1. Adeno associated virus (AAV) particle, comprising a modified capsid, wherein said modified capsid comprises at least one modification selected from the removal of a natural binding site in said capsid, and the introduction of a ligand binding site into said capsid.

2. The AAV particle according to claim 1, wherein said AAV is selected from AAV1, AAV2, AAV3, AAV4, AAV5, AAV6, AAV7, AAV8, AAV9, AAV10, AAV11, and AAV12.

3. The AAV particle according to claim 1 or 2, wherein at least one protein in said capsid is modified, preferably wherein said at least one protein is VP1, VP2, and/or VP3.

4. The AAV particle according to any one of claims 1 to 3, wherein said modification of said capsid comprises the removal of at least one natural binding site and the introduction of at least one ligand binding site.

5. The AAV particle according to any one of claims 1 to 4, wherein said natural binding site is a natural binding site that enables binding to heparan sulfate proteoglycans, and is preferably removed by replacing at least one of arginines 585 and 588 of VP1 and/or an analogous arginine in VP2 or VP3 with a different amino acid, such as alanine.

6. The AAV particle according to any one of claims 1 to 5, wherein said ligand binding site is a ligand binding site that enables the covalent attachment of a ligand, and is preferably selected from a benzylguanine group, a benzylcytosine group, a chloroalkane group, an azide group, a phosphine, or combinations thereof.

7. The AAV particle according to claim 6, further comprising a ligand that is attached to said benzylguanine group, said benzylcytosine group, said chloroalkane group, said azide group and/or said phosphine in particular a HaloTag™, a SNAP-tag™, or a CLIP-tag™, an azide or a phosphine.

8. The AAV particle according to claim 6 or 7, wherein said ligand is selected from a protein ligand, a toxin subunit, a lectin, an adhesion factor, an antibody, a peptide, and a gene editing nuclease.

9. A method of producing an improved adeno associated virus (AAV) particle, comprising the step of introducing at least one modification into a capsid of said AAV, preferably wherein said modification comprises introducing at least one ligand binding site into said capsid, optionally wherein a natural binding site in said capsid is removed.

10. A pharmaceutical composition, comprising the AAV particle according to any one of claims 1 to 8, together with at least one pharmaceutically acceptable carrier and/or diluent.

11. The AAV particle according to any one of claims 1 to 8 or the pharmaceutical composition according to claim 10 for use in the treatment of a disease, wherein said disease is preferably one that can be treated by gene therapy, such as cancer, an inherited monogenic disease, a genetic skin disease, an infectious disease, type I diabetes, and wound healing.

12. A method for treating a disease that can be treated by gene therapy, comprising administering the AAV particle according to any one of claims 1 to 8 or the pharmaceutical composition according to claim 10 to a subject in need thereof, wherein said disease is preferably one that can be treated by gene therapy, such as cancer, an inherited monogenic disease, a genetic skin disease, an infectious disease, type I diabetes, and wound healing.

13. Use of the AAV particle according to any one of claims 1 to 8 for the transfection of a cell, for example as a gene delivery tool.

14. The use of the AAV particle according to claim 13, wherein said use is for cosmetic purposes.
